# EUROPEAN PATENT SPECIFICATION

(11) **EP 1 734 911 B1**
(45) Date of publication and mention of the grant of the patent: **06.04.2016**
(21) Application number: 05732222.4
(22) Date of filing: 07.04.2005
(51) Int. Cl.: A61F 13/56, A61F 13/82

(54) **ABSORBENT ARTICLE WITH FOLDED SIDE FLAP PORTIONS**
SAUGFÄHIGER ARTIKEL MIT GEFALTETEN SEITENKLAPPENABSCHNITTEN
ARTICLE ABSORBANT AVEC RABATS LATERAUX PLIES

(30) Priority: 13.04.2004 GB 0408241
(43) Date of publication of application: 27.12.2006
(73) Proprietor: SCA HYGIENE PRODUCTS AB, 405 03 Göteborg (SE)
(72) Inventor: DREVIK, Solgun, S-435 35 Mölnlycke (SE); STRIDFELDT, Chatrine, S-436 58 Hovås (SE)
(74) Representative: Romare, Laila Anette
(86) International application number: PCT/SE2005/000505
(87) International publication number: WO 2005/099645

(56) References cited:
- EP-A1- 0 638 303
- WO-A1-96/29968
- WO-A1-99/01094
- GB-A- 2 284 767
- US-A- 5 807 367

## Description

### TECHNICAL FIELD

The invention refers to an absorbent article, such as a sanitary napkin, female or male incontinence article, panty liner or a hygiene product, comprising a backsheet covered at least partly by a topsheet, which sanitary napkin extends in a longitudinal direction and in a lateral direction. The sanitary napkin is delimited by a first and a second longitudinally extending side edge and a first and a second laterally extending short edges. The invention also refers to a method for the manufacture of the absorbent article.

### BACKGROUND ART

Absorbent articles such as sanitary napkins, female or male incontinence articles, panty liners and hygiene product, are well known for protecting undergarments from bodily exudates, for example, menstrual fluids. It is important that the sanitary napkin is well fitted and secured against the user's body such that the sanitary napkin stays in place during use, i.e. both when the user is still and moves, such that no leakage occurs. It is known to use adherent means for securing the sanitary napkin against the user's skin. The adherent means comprises chemical components that stick to the user's skin. In order for the sanitary napkin to have a good fit, the adherent means must not stick to the inner thighs of the user, since this would cause the sanitary napkin to move relative the groin area when the legs are moving. Therefore, the adhering means should stick to the groin area and parts of the lower abdomen.

EP 788338 teaches a self supported sanitary napkin with an adhesive placed on the topsheet along longitudinal side edges. The adhesive has a special composition that makes the sanitary napkin to adhere to the wearer's skin, but that does not adhere to the wearer's pubic hair. This enables a relatively pain-free removal of the article.

There are several disadvantage with the sanitary napkin disclosed in EP 788338. One disadvantage is that the adhesive is applied directly on the topsheet. This may cause soiling of the product during manufacturing of the sanitary napkin. Another drawback is that the adhesive applied on the topsheet diminishes the possible liquid transport since the adhesive blocks and clogs the structure of the topsheet. A further disadvantage is that the adhesive may run through the topsheet to an underlying absorbent layer, which causes a lesser absorbent ability due to less available absorbent volume.

There is thus a need for an improved absorbent article with a better fit to the wearer's body and an improved method for manufacturing the absorbent article.

### DISCLOSURE OF INVENTION

The object of the present invention is to remedy the above problems. The invention thus refers to an absorbent article such as a sanitary napkin, female or male incontinence article, panty liner or a hygiene product, comprising a backsheet covered at least partly by a topsheet. The absorbent article refers to small hygiene articles intended to be worn in an undergarment and do not refer to a diaper or larger incontinence articles.

The absorbent article extends in a longitudinal direction and in a lateral direction. The absorbent article is delimited by a first and a second longitudinally extending side edge and a first and a second opposing laterally extending short edge. The invention is characterised in that the backsheet comprises a first flap portion extending at least partly along the first side edge and folded over the first side edge and over a part of the topsheet. The backsheet further comprises a second flap portion extending at least partly along the second side edge and folded over the second side edge and over a part of the topsheet. The first and second flap portions are at least partly covered with an adherent means.

One advantage with the absorbent article according to the invention is that the folded over side flap portions adheres better to the groin area of the user than the absorbent article according to prior art because of the curvature of the flap portions.

One further advantage is that the folded over flap portions gives the benefits of a god positioning of the adherent means. The adherent means follows the curvature of the folded over flap portions, which extends over at least the longitudinally extending side edges and then over the side margins of the topsheet. The folded over flap portions constitute that part of the absorbent article that during use is placed against the user's groin area and not to the inner thighs of the user. Furthermore, dependent on the extension of the side flap portions over the topsheet, the flap portions and thus the adherent means may adhere also to parts of the lower abdomen.

The topsheet may be a conventional topsheet that is liquid permeable and soft for the skin, for example a nonwoven. However, the topsheet may also comprise absorptive means, i.e. the topsheet may be an absorbent layer comprising for example absorbent fibers.

The adherent means advantageously has an adhesive force to human skin lesser than to the respective flap portion material. This means that the adherent means or parts of the adherent means do not stay on the skin of the user when the absorbent article is removed after use.

In one embodiment the adherent means comprises an adhesive. For example, a skin friendly compound as presented in EP 0788338. One advantage of this embodiment is that the adherent means do not stick to pubic hair. The adhesive may also be a skin friendly glue or another suitable adhesive such as hydrogel. The hydrogel is made from a material which does not irritate the mucous membranes of the user and can comprise polymers based on vinyl alcohol, polyacrylate, polymethacrylate, polymethylene oxide, polysaccharide, acrylamide, vinyl pyrrolidone, or urethane, and mixtures, copolymers and derivates thereof. It is also possible to use protein-based hydrogels.

In another embodiment of the invention, the adherent means comprises a non-adhesive. Here non-adhesive means a compound or a structure that do not stick to the skin of the user due to adhesive forces, but adhere to the skin due to for example frictional forces or vacuum force or another suitable force. However, common for all adherent means are that they stick to the flap portions in a higher degree than to the user's skin.

In one embodiment of the invention, the first flap portion and the second flap portion comprise joined parts of the topsheet and the backsheet. The topsheet may thus have the same extension as the backsheet, at least in that part of the absorbent article that constitute the first flap portion and the second flap portion.

In one embodiment of the invention, the first flap portion and the second flap portion constitute separate parts attached to the backsheet. The flap portions may be formed of the same or a different material than the backsheet and may be attached to the backsheet by any suitable means, for example gluing or welding.

The first flap portion and the second flap portion is attached to the topsheet in at least one point by fastening means, such that channels are formed between each flap portion and the top sheet. Each flap portion is preferably attached in a number of points which gives a number of channels between each point. The attachment may be done by gluing or welding (for example ultrasonic welding) or by another suitable method. The "attachment in points" refers to fastening means such as a seam or the like, which may have any suitable form for example lumped or a curved line or a straight line.

In one embodiment of the invention the absorbent article comprises an absorbent layer sandwiched between the backsheet and the topsheet. Here "sandwiched" refers to the forming of a lamellar structure.

In one embodiment of the invention, the absorbent article comprises a flap comprising the first flap portion and the second flap portion and a third flap portion extending along the first lateral short edge, connecting the first flap portion with the second flap portion. The flap may be attached to the topsheet in the same way as been described above.

In another embodiment of the invention, the absorbent article comprises a flap extending along the periphery of the entire sanity napkin. The flap thus comprises the first flap portion, the second flap portion, the third flap portion and a fourth flap portion extending along the second lateral short edge, connecting the first flap portion with the second flap portion. The flap may be attached to the topsheet as been described above. However, when the absorbent article is intended for use as an incontinence article for men, it is advantageous if at least the fourth flap portion may be lifted from the topsheet and placed behind the user's scrotum. In this embodiment the flap is folded over the edges of the absorbent article and stays in place due to the lesser inner perimeter of the flap than the outer perimeter of the absorbent article, i.e. the perimeter described by the edges.

The invention also refers to a method for manufacturing an absorbent article, where the method comprises the steps of;
- sandwiching the topsheet and the backsheet;
- covering the first and second flap portions at least partly with an adherent means;
- folding the first flap portion over the first side edge and over a part of the topsheet, and;
- folding the second flap portion over the second side edge and over a part of the topsheet.

The method is advantageous because the adherent means are applied to the flap or flap portions when the absorbent article is in an unfolded state, which minimises the risk of the adherent means soiling the topsheet during production. Furthermore, the backsheet is preferably made of a liquid impermeable material (for example a plastic film) that has a smooth surface. The adherent means has a better adherent force to such material than to a soft and textured topsheet.

The absorbent article may have a circular form, oval form, triangular form, rectangular form, hour-glass shaped form, or any combination of said forms.

The flap or the flap portions may comprise elastic means for forming the flap portions. The elastic means may also be used to form the absorbent article by exerting a contracting force on the absorbent article. The elastic means may be placed in the flap portions as pre-tensioned strings along the peripheral extension of the flap portions. Alternatively, the flap portions per se may be of an elastic material. The elastic means then contract parts of the flap portions such that openings to the channels are reinforced. The elastic means may thus advantageously be placed in the flap portions at lest between two attachment points. The elastic means are advantageously placed in the flap portions before the flap portions are folded over the top sheet. The elastic means may then be secured in position when the flap portions are attached or secured to the topsheet. The elastic means gives an absorbent article that is flexible and adaptive in regard to the movement of the user.

One further advantage with the invention is that the flap or flap portion creates a distance between the rest of the article and the user's body that changes dependent on the user's movement and position. For example, if the user is using the article without any undergarment or with loosely fitted garments, the flap or flaps is attached to the user's body and a distance is created between the topsheet and the user's lower abdomen. The distance gives a better ventilation than previously known product that are applied in close contact with the lower abdomen. Since the distance changes with the movement and position, for example if the user goes from standing to sitting, different air pressures are created between the article and the lower abdomen. The difference in air pressure creates an air flow in the space between the article and the lower abdomen, which gives an advantageous ventilation of the user's lower abdomen.

In the case where the flap or flap portion extends on all sides, i.e. peripherally, the flap or flap portion advantageously acts as a bellows when the distance is changed, ensuring good ventilation.

The absorbent article may be packed in a wrapping enveloping the entire absorbent article. For example in a sealed bag-like container or a sheet wrapped around the absorbent article. The wrapping may also have an extension covering only the upper part of the absorbent article, i.e. the flap or flap portion and the topsheet.

The wrapping is advantageously intended to cover and protect the adherent flap or flap portion and in some cases also the topsheet. The wrapping is either made from a material that does not adhere to the adherent means or only adheres in such a way that the adherent means do not stick to the wrapping upon removal of the wrapping. This may be done also by treating the wrapping with a release agent.

The advantage of using a wrapping as described above is that the topsheet and the flap or flap portion is hygienically protected and thus offers a hygienic product to the user.

### BRIEF DESCRIPTION OF DRAWINGS

The invention will below be explained further with reference to a number of drawings, where;
Fig. 1 schematically shows a top view of a sanitary napkin according to one embodiment of the invention;
Fig. 2 schematically shows a cross-sectional sides view along line A-A in fig. 1, according to a first embodiment of the invention;
Fig. 3 schematically shows a cross-sectional side view along line A-A in fig. 1, according to a second embodiment of the invention, where the sanitary napkin is in an unfolded state;
Fig. 4 schematically shows a cross-sectional side view along line A-A in fig. 1, according to the second embodiment in fig. 3, where the sanitary napkin is in a folded state;
Fig. 5 schematically shows a cross-sectional side view along line A-A in fig. 1, according to a third embodiment of the invention, where the sanitary napkin is in an unfolded state;
Fig. 6 schematically shows a cross-sectional side view along line A-A in fig. 1, according to the third embodiment in fig. 5, where the sanitary napkin is in a folded state;
Fig. 7 schematically shows a cross-sectional side view along line A-A in fig. 1, according to a fourth embodiment of the invention, where the sanitary napkin is in an unfolded state;
Fig. 8 schematically shows a cross-sectional side view along line A-A in fig. 1, according the fifth embodiment in fig. 7, where the sanitary napkin is in an unfolded state;
Fig. 9 schematically shows a top view of a sanitary napkin according to a sixth embodiment of the invention, and where;
Fig. 10 schematically shows a top view of a sanitary napkin according a seventh embodiment of the invention.

### MODES FOR CARRYING OUT THE INVENTION

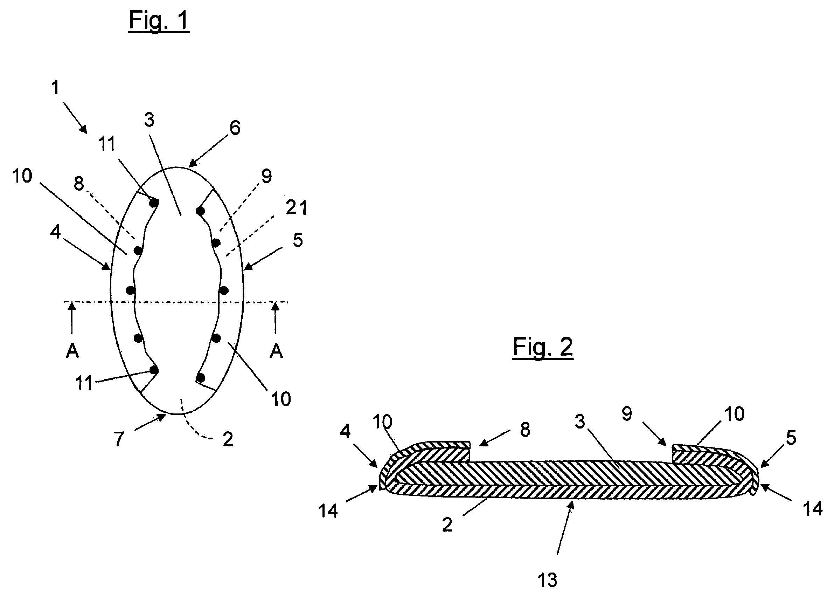

Fig. 1 schematically shows a top view of a sanitary napkin 1 according to one embodiment of the invention. The sanitary napkin 1 comprises a backsheet 2 covered at least partly by a topsheet 3, which sanitary napkin 1 extends in a longitudinal direction and in a lateral direction, the sanitary napkin is delimited by a first and a second longitudinally extending side edge 4, 5 and two opposing and essentially laterally extending short edges 6, 7. The backsheet comprises a first flap portion 8 extending at least partly along the first side edge 4 and folded over the first side edge 4 and over the topsheet 3, and a second flap portion 9 extending at least partly along the second side edge 5 and folded over the second side 5 edge and over the topsheet 3. The first and second flap portions 8, 9 are at least partly covered with an adherent means 10 with an adhesive force to human skin lesser than to the respective flap portion 8, 9 material.

The flap portions 8, 9 are intended to adhere to the body of the user such that the sanitary napkin stays in place during use. Furthermore, the flap portions 8, 9 advantageously form side leakage barriers, preventing bodily fluids from leaking along the first and second edges 4, 5 .

Figure 1 shows that the flap portions 8, 9 are fastened to the topsheet with fastening means 11. The fastening means 11 may comprise an adhesive such as glue or may be formed by welding or another suitable fastening means. Figure 1 shows that the fastening means 11 fastens the flap portions 8, 9 to the topsheet 3 at several points along the extension of each flap portions 8, 9. The fastening means are not restricted to such positions, but the positions may be varied along the entire extension of the flap portions 8, 9. The fastening means 11 may vary in shape and size. For example, the fastening means may be in the form of seams extending along any direction of the flap portion 8 or 9, i.e. along the longitudinal direction, the lateral direction or a combination of the both. The seams may also be curved or straight.

The parts of the flap portions 8, 9 between the fastening means 11 are loosely fit to the topsheet 3 and thus create channels 21 between each flap portion 8, 9 and the topsheet 3. The channels 21 provide a better liquid transport from the centre of the napkin towards the longitudinally extending first and second side edges 4, 5.

Fig. 2 schematically shows a cross-sectional side view along line A-A in fig. 1, according to a first embodiment of the invention. In the first embodiment the topsheet 3 comprises an absorbent material for absorbing fluids when the article is in use. The topsheet 3 has an extension essentially similar to the extension of the backside 13. In an unfolded state, the topsheet 3 has an extension similar to the extension of the backsheet 2, except for the flap portions 8, 9.

Fig. 2 shows that the adherent means 10 are applied on the flap portions 8, 9 covering the entire flap portion extending from a backside 13 of the article, over the curved parts 14 coinciding with the side edges 4, 5, and over the topsheet. The adherent means 10 may however be applied to the flap portions 8, 9 in a discontinuous pattern. The adherent means 10 shall not be applied to the flap portions 8, 9 such that the adherent means 10 adheres to the undergarment when in use. This means that, in figure 2, the adherent means 10 may not extend beyond the point where the curved parts 14 transitions into the flat backside 13 of the backsheet 2.

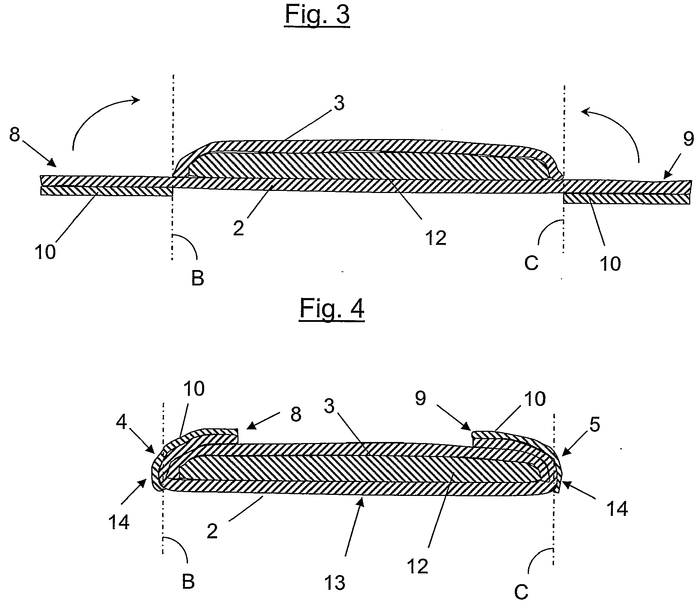

Fig. 3 schematically shows a cross-sectional side view along line A-A in fig. 1, according to a second embodiment of the invention, where the sanitary napkin 1 is in an unfolded state. Figure 3 shows that the adherent means have been applied on the flap portions 8, 9 when the flap portions 8, 9 are unfolded, which has the benefit of minimising the risk of soiling the topsheet 3 when applying the adherent means 10. Figure 3 shows lines B and C, which delimit the backsheet into the backside 13 and the first and second flap portion 8, 9. In figure 3 lines B and C are placed where the topsheet 3 meets the backsheet 2. The lines B and C have thus been placed at a position marking a folding position over which the first and second flap portions 8, 9 may be folded.

When the adherent means 10 are applied, the flap portions 8, 9 are folded in the directions as indicated by the arrows shown in figure 3. Figure 3 also show an absorbent layer sandwiched between the topsheet 3 and the backsheet 2. In this embodiment, the topsheet 3 may be a liquid transport layer and/or a liquid spreading layer, without any substantial liquid absorbing features. However, the topsheet 3 may be in the form of a liquid absorbent layer with the same or different liquid absorbent properties as the underlying absorbent layer 12.

Fig. 4 schematically shows a cross-sectional side view along line A-A in fig. 1, according the second embodiment in fig. 3 and in a folded state. As seen in figure 4, the topsheet is joined to the backsheet along the periphery of the absorbent body 12 at least along the longitudinally extending side edges 4, 5. The flap portions 8, 9 are then folded over the joined parts along folding lines crossing lines B and C, and over parts of the topsheet 3.

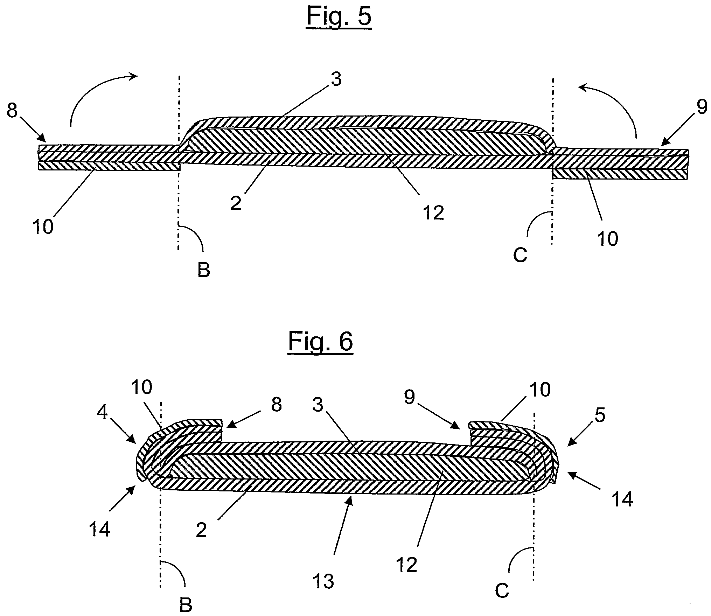

Fig. 5 schematically shows a cross-sectional side view along line A-A in fig. 1, according to a third embodiment of the invention, where the sanitary napkin is in an unfolded state. Figure 5 shows that the topsheet 3 extends over the absorbent body 12 and over the entire extension of the backsheet 2. In figure 5 lines B and C are placed where the topsheet 3 meets the backsheet 2. The lines B and C have been placed at a position marking a suitable folding position over which the first and second flap portions 8, 9 may be folded. The third embodiment is in all other considerations equivalent with the second embodiment.

Fig. 6 schematically shows a cross-sectional side view along line A-A in fig. 1, according the third embodiment in fig. 5 and in a folded state. As seen in figure 6, the topsheet is joined to the backsheet along the periphery of the sanitary napkin 1 at least along the longitudinally extending first and second side edges 4, 5. The flap portions 8, 9 are then folded over the joined parts along folding lines crossing lines B and C, and over parts of the topsheet 3.

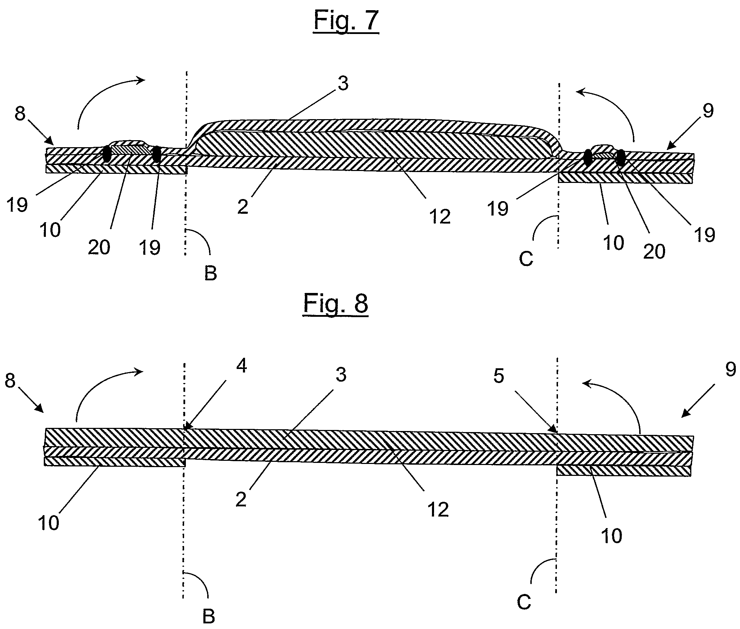

Fig. 7 schematically shows a cross-sectional side view along line A-A in fig. 1, according to a fourth embodiment of the invention, where the sanitary napkin is in an unfolded state. The third embodiment is in all considerations equivalent with the third embodiment, but with the difference that an additive 20 has been placed in each flap portion 8, 9, between the topsheet and the backsheet 2. The additive 20 is secured by seams 19, placed on either side of the additive. The additive may be a lotion, a deodorant or anti odour compound, an antibacterial compound, an extra absorptive compound, or another suitable active or inactive component. The compound may diffuse through the topsheet in the first and second flap portions 8, 9, or the bodily fluids may diffuse through the topsheet 3 to the additive in the pocket created by the flap portions 8, 9. The seams may be done by welding or gluing or another suitable method. One advantage of the embodiment is that the psychological advantage of not placing an additive directly on the skin of the user. One further advantage is that the additive will not cause irritation to the skin of the user nor will it cause friction between the skin and the absorbent article.

As in figure 6, the first and second side flap portions 8, 9 are folded along folding lines crossing lines B and C.

Fig. 8 schematically shows a cross-sectional side view along line A-A in fig. 1, according to a fifth embodiment of the invention, where the sanitary napkin is in an unfolded state. Fig. 8 shows that the topsheet 3 is in the form of an absorbent layer that in the lateral direction and along the longitudinally extending first and second side edges 4, 5 has the same extension as the backsheet 2. The first and second side flap portions 8, 9 are folded along folding lines crossing lines B and C. In fig. 8, lines B and C are placed at a suitable distance from the joined edge of the topsheet 3 and the backsheet 2. On each of the first and second flap portions 8, 9, the distance coincides with the lateral extension of the adherent means 10.

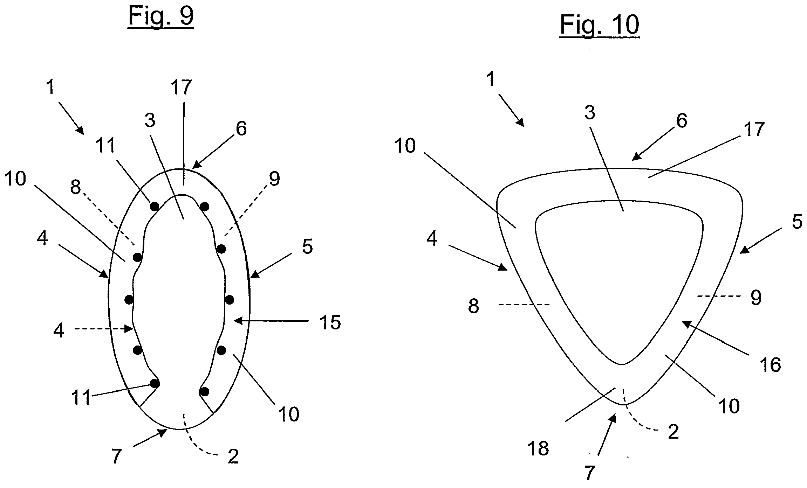

Fig. 9 schematically shows a top view of a sanitary napkin according to a sixth embodiment of the invention. Fig. 9 shows a sanitary napkin 1 comprising a flap 15 comprising the first flap portion 8 and the second flap portion 9 and a third flap portion 17 extending along the first lateral short edge 6, connecting the first flap portion 8 with the second flap portion 9.

Figure 9 shows that the flap 15 extends along the first and second side edges 4, 5 and the first short edge 6, and is folded over the topsheet 3. The entire flap 15 comprises a part of the backsheet 2 and may comprise parts of the topsheet 3. All embodiments described in connection to figures 2-8 are possible together with the embodiment shown in fig. 9. The adherent means 10 is thus applied on the flap 15, on the backsheet 2, when the flap 15 is in an unfolded position.

Fig. 10 schematically shows a top view of a sanitary napkin 1 according a seventh embodiment of the invention. Fig. 10 shows a sanitary napkin 1 comprising a flap 16 extending along the entire periphery of the sanity napkin. The flap 16 comprises the first flap portion 8, the second flap portion 9, the third flap portion 17 and a fourth flap portion 18 extending along the second lateral short edge 7, connecting the first flap portion 8 with the second flap portion 9.

Figure 10 shows that the flap 16 extends along the first and second side edges 4, 5 and the first and second short edges 6, 7. The flap is folded over at least a part of the topsheet 3. The entire flap 16 comprises a part of the backsheet 2 and may comprise parts of the topsheet 3. All embodiments described in connection to figures 2-8 are possible together with the embodiment shown in fig. 10. The adherent means 10 is thus applied on the flap 16, on the backsheet 2, when the flap 16 is in an unfolded position.

Figure 10 shows that the sanitary napkin has an essentially triangular geometry. The second short edge 7 is here essentially shorter than the first short edge 6. The seventh embodiment is especially advantageous for use as an incontinence article for men. During use of the article, the fourth flap portion 18 is then advantageously placed behind the user's scrotum. Figure 10 shows that the flap 16 is not attached to the topsheet. This is because the, on all sides extending, flap 16 has a smaller inner periphery than the periphery of the sanitary napkin 1, which gives that the flap 16 automatically is kept in position after being folded over the edges 4-7. However, the flap 16 may be attached to the topsheet as described in connection to figures 1-9.

The invention is not restricted to the above embodiments, but may be varied within the scope of the claims. For example, the sanitary napkin may have any suitable form, for example hour-glass-shaped, oval (as in figures 1-9), triangular (as in figure 10), rectangular, trapetzoid, or any other suitable geometry. Regardless of the geometry, the flap may comprise the first and second flap portion and/or the third flap portion and/or the fourth flap portion.

Furthermore, the adherent means need not cover the entire flap or flap portions, but may be applied on the flap or flap portions in an optional pattern, for example dots, stripes etc.

The flaps or flap portions may be discontinuous, i.e. comprise a number of flap portion segments.

## Claims

1. An absorbent article (1) such as a sanitary napkin, female or male incontinence article, panty liner or a hygiene product, comprising a backsheet (2) covered at least partly by a topsheet (3), an absorbent article (1) extends in a longitudinal direction and in a lateral direction, the absorbent article (1) is delimited by a first and a second longitudinally extending side edge (4, 5) and a first and a second laterally extending short edge (6, 7), **characterised in that**; the backsheet (2) comprises a first flap portion extending at least partly along the first side edge (4) and folded over the first side edge (4) and over a part of the topsheet (3); the backsheet (2) further comprises a second flap portion (9) extending at least partly along the second side edge (5) and folded over the second side edge (5) and over a part of the topsheet (3), wherein the first and second flap portions (8, 9) are at least partly covered with an adherent means (10) and the first flap portion (8) and the second flap portion (9) are attached to the topsheet (3) in at least one point by fastening means (11), such that channels (21) are formed between each flap portion (8, 9) and the top sheet (3).

2. An absorbent article (1) according to claim 1, **characterised in that** the adherent means has an adhesive force to human skin lesser than to the respective flap portion material.

3. An absorbent article (1) according to claim 2, **characterised in that** the adherent means (10) comprises an adhesive.

4. An absorbent article (1) according to any one of claims 1-3, **characterised in that** the first flap portion (8) and the second flap portion (9) comprise joined parts of the topsheet (3) and the backsheet (2).

5. An absorbent article (1) according to any one of claims 1-3, **characterised in that** the first flap portion (8) and the second flap portion (9) constitute separate parts attached to the backsheet (2).

6. An absorbent article (1) according to any one of the preceding claims, **characterised in that** the absorbent article (1) comprises an absorbent layer (12) sandwiched between the backsheet (2) and the topsheet (3).

7. An absorbent article (1) according to any one of the preceding claims, **characterised in that** the absorbent article (1) comprises a flap (15, 16) comprising the first flap portion (8) and the second flap portion (9) and a third flap portion (17) extending along the first lateral short edge (6), connecting the first flap portion (8) to the second flap portion (9).

8. An absorbent article (1) according to claim 7, **characterised in that** the absorbent article (1) comprises a flap (16) extending along the periphery of the entire absorbent article (1), comprising the first flap portion (8), the second flap portion (9), the third flap portion (17) and a fourth flap portion (18) extending along the second lateral short edge, connecting the first flap portion (8) to the second flap portion (9).

9. A method for manufacturing an absorbent article (1), comprising a backsheet (2) covered at least partly by a topsheet (3), which absorbent article (1) extends in a longitudinal direction and in a lateral direction, the absorbent article is delimited by a first and a second longitudinally extending side edge (4, 5) and a first and second laterally extending short edge (6, 7), wherein the backsheet (2) comprises a first flap portion (8) extending at least partly along the first side edge (4) a second flap portion (9) extending at least partly along the second side edge (5), wherein the method comprises the steps of;
- sandwiching the topsheet (3) and the backsheet (2);
- covering the first and second flap portions (8, 9) at least partly with an adherent means (10);
- folding the first flap portion (8) over the first side edge (4) and over a part of the topsheet (3), and;
- folding the second flap portion (9) over the second side edge and over a part of the topsheet.
- Attaching the first flap portion (8) to the topsheet (3) in at least one point, forming channels (21) between the first flap portion (8) and the top sheet (3).
- Attaching the second flap portion (9) to the topsheet (3) in at least one point, forming channels (21) between the second flap portion (9) and the top sheet (3).

10. A method according to claim 9, **characterised in that** the adherent means has an adhesive force to human skin lower than to the respective flap portion (8, 9) material.

11. A method according to claim 9 or 10, **characterised in that** parts of the topsheet (3) and the backsheet (2) are joined forming the first flap portion (8) and the second flap portion (9).

12. A method according to any one of claims 9-11, **characterised in that** the first flap portion (8) and the second flap portion (9) constitute separate parts attached to the backsheet (2).

13. A method according to any one of claims 9-12, **characterised in that** an absorbent layer (12) is sandwiched between the backsheet (2) and the topsheet (3).

14. A method according to any one of claims 9-13, **characterised in that** the absorbent article (1) comprises a flap (15, 16) comprising the first flap portion (8) and the second flap portion (9) and a third flap portion (17) extending along the first lateral short edge (6), connecting the first flap portion (8) with the second flap portion (9), where the flap (15, 16) is folded over the edges (4-6) and parts of the topsheet (3).

15. A method according to claim 14, **characterised in that** the absorbent article (1) comprises a flap (16) extending along the periphery of the entire absorbent article (1), comprising the first flap portion (8), the second flap portion (9), the third flap portion (17) and a fourth flap portion (18) extending along the second lateral short edge, connecting the first flap portion (8) with the second flap portion (9), where the flap (16) is folded over the edges (4-7) and parts of the topsheet (3).

## Patentansprüche

1. Saugfähiger Artikel (1), wie etwa eine Damenbinde, ein Inkontinenzartikel für Frauen oder Männer, eine Slipeinlage oder ein Hygieneprodukt, umfassend eine zumindest teilweise von einer Deckschicht (3) bedeckte Rückschicht (2), wobei ein saugfähiger Artikel (1) sich in eine Längsrichtung und eine seitliche Richtung erstreckt, wobei der saugfähige Artikel (1) durch einen ersten und einen zweiten längs verlaufenden Seitenrand (4, 5) und einen ersten und einen zweiten seitlich verlaufenden kurzen Rand (6, 7) abgegrenzt ist, **dadurch gekennzeichnet dass** die Rückschicht (2) einen ersten Laschenabschnitt umfasst, der sich zumindest teilweise entlang des ersten Seitenrands (4) erstreckt und über den ersten Seitenrand (4) und über einen Teil der Deckschicht (3) gefaltet ist; die Rückschicht (2) umfasst ferner einen zweiten Laschenabschnitt (9), der sich zumindest teilweise entlang des zweiten Seitenrands (5) erstreckt und über den zweiten Seitenrand (5) und über einen Teil der Deckschicht (3) gefaltet ist, wobei der erste und der zweite Laschenabschnitt (8, 9) zumindest teilweise mit einem anhaftenden Mittel (10) überzogen sind und der erste Laschenabschnitt (8) und der zweite Laschenabschnitt (9) an zumindest einem Punkt durch Befestigungsmittel (11) an der Deckschicht (3) angebracht sind, sodass zwischen jedem Laschenabschnitt (8, 9) und der Deckschicht (3) Kanäle (21) ausgebildet sind.

2. Saugfähiger Artikel (1) nach Anspruch 1, **dadurch gekennzeichnet, dass** das anhaftende Mittel eine schwächere Haftkraft an menschlicher Haut als an dem jeweiligen Laschenabschnittmaterial aufweist.

3. Saugfähiger Artikel (1) nach Anspruch 2, **dadurch gekennzeichnet, dass** das anhaftende Mittel (10) ein Klebemittel umfasst.

4. Saugfähiger Artikel (1) nach einem der Ansprüche 1-3, **dadurch gekennzeichnet, dass** der erste Laschenabschnitt (8) und der zweite Laschenabschnitt (9) zusammengefügte Teile der Deckschicht (3) und der Rückschicht (2) umfassen.

5. Saugfähiger Artikel (1) nach einem der Ansprüche 1-3, **dadurch gekennzeichnet, dass** der erste Laschenabschnitt (8) und der zweite Laschenabschnitt (9) separate, an der Rückschicht (2) angebrachte Teile darstellen.

6. Saugfähiger Artikel (1) nach einem der vorgehenden Ansprüche, **dadurch gekennzeichnet, dass** der saugfähige Artikel (1) eine Saugschicht (12) umfasst, die zwischen der Rückschicht (2) und der Deckschicht (3) angeordnet ist.

7. Saugfähiger Artikel (1) nach einem der vorhergehenden Ansprüche, **dadurch gekennzeichnet, dass** der saugfähige Artikel (1) eine Lasche (15, 16) umfasst, welche den ersten Laschenabschnitt (8) und den zweiten Laschenabschnitt (9) sowie einen sich entlang des ersten seitlichen kurzen Rands (6) erstreckenden dritten Laschenabschnitt (17), der den ersten Laschenabschnitt (8) mit dem zweiten Laschenabschnitt (9) verbindet, umfasst.

8. Saugfähiger Artikel (1) nach Anspruch 7, **dadurch gekennzeichnet, dass** der saugfähige Artikel (1) eine Lasche (16) umfasst, welche sich entlang des gesamten Umfangs des saugfähigen Artikels (1) erstreckt und den ersten Laschenabschnitt (8), den zweiten Laschenabschnitt (9), den dritten Laschenabschnitt (17) und einen sich entlang des zweiten seitlichen kurzen Rands erstreckenden vierten Laschenabschnitt (18), der den ersten Laschenabschnitt (8) mit dem zweiten Laschenabschnitt (9) verbindet, umfasst.

9. Verfahren zur Herstellung eines saugfähigen Artikels (1), umfassend eine zumindest teilweise von einer Deckschicht (3) bedeckte Rückschicht (2), wobei der saugfähige Artikel (1) sich in eine Längsrichtung und eine seitliche Richtung erstreckt, wobei der saugfähige Artikel durch einen ersten und einen zweiten längs verlaufenden Seitenrand (4, 5) und einen ersten und einen zweiten seitlich verlaufenden kurzen Rand (6, 7) abgegrenzt ist, wobei die Rückschicht (2) einen ersten Laschenabschnitt (8) umfasst, der sich zumindest teilweise entlang des ersten Seitenrands (4) erstreckt, einen zweiten Laschenabschnitt (9), der sich zumindest teilweise entlang des zweiten Seitenrands (5) erstreckt, umfasst, wobei das Verfahren die folgenden Schritte umfasst;
- Übereinanderlegen der Deckschicht (3) und der Rückschicht (2);
- Überziehen des ersten und zweiten Laschenabschnitts (8, 9) zumindest teilweise mit einem anhaftenden Mittel (10);
- Falten des ersten Laschenabschnitts (8) über den ersten Seitenrand (4) und über einen Teil der Deckschicht (3), und;
- Falten des zweiten Laschenabschnitts (9) über den zweiten Seitenrand und über einen Teil der Deckschicht.
- Befestigen des ersten Laschenabschnitts (8) an zumindest einem Punkt an der Deckschicht (3), wodurch Kanäle (21) zwischen dem ersten Laschenabschnitt (8) und der Deckschicht (3) ausgebildet werden.
- Befestigen des zweiten Laschenabschnitts (9) an zumindest einem Punkt an der Deckschicht (3), wodurch Kanäle (21) zwischen dem zweiten Laschenabschnitt (9) und der Deckschicht (3) ausgebildet werden.

10. Verfahren nach Anspruch 9, **dadurch gekennzeichnet, dass** das anhaftende Mittel eine geringere Haftkraft an menschlicher Haut als an dem jeweiligen Laschenabschnittmaterial (8, 9) aufweist.

11. Verfahren nach Anspruch 9 oder 10, **dadurch gekennzeichnet, dass** Teile der Deckschicht (3) und der Rückschicht (2) zusammengefügt werden und den ersten Laschenabschnitt (8) und den zweiten Laschenabschnitt (9) ausbilden.

12. Verfahren nach einem der Ansprüche 9-11, **dadurch gekennzeichnet, dass** der erste Laschenabschnitt (8) und der zweite Laschenabschnitt (9) separate, an der Rückschicht (2) angebrachte Teile darstellen.

13. Verfahren nach einem der Ansprüche 9-12, **dadurch gekennzeichnet, dass** eine Saugschicht (12) zwischen der Rückschicht (2) und der Deckschicht (3) angeordnet ist.

14. Verfahren nach einem der Ansprüche 9-13, **dadurch gekennzeichnet, dass** der saugfähige Artikel (1) eine Lasche (15, 16) umfasst, welche den ersten Laschenabschnitt (8) und den zweiten Laschenabschnitt (9) sowie einen sich entlang des ersten seitlichen kurzen Rands (6) erstreckenden dritten Laschenabschnitt (17), der den ersten Laschenabschnitt (8) mit dem zweiten Laschenabschnitt (9) verbindet, umfasst, wobei die Lasche (15, 16) über die Ränder (4-6) und Teile der Deckschicht (3) gefaltet wird.

15. Verfahren nach Anspruch 14, **dadurch gekennzeichnet, dass** der saugfähige Artikel (1) eine Lasche (16) umfasst, welche sich entlang des gesamten Umfangs des saugfähigen Artikels (1) erstreckt und den ersten Laschenabschnitt (8), den zweiten Laschenabschnitt (9), den dritten Laschenabschnitt (17) und einen sich entlang des zweiten seitlichen kurzen Rands erstreckenden vierten Laschenabschnitt (18), der den ersten Laschenabschnitt (8) mit dem zweiten Laschenabschnitt (9) verbindet, umfasst, wobei die Lasche (16) über die Ränder (4-7) und Teile der Deckschicht (3) gefaltet wird.

## Revendications

1. Article absorbant (1) tel qu'une serviette hygiénique, un article pour l'incontinence féminine ou masculine, un protège-slip ou un produit d'hygiène, comprenant une feuille de fond (2) couverte au moins partiellement par une feuille de dessus (3), un article absorbant (1) s'étendant dans une direction longitudinale et dans une direction latérale, l'article absorbant (1) étant délimité par des premier et second bords latéraux s'étendant longitudinalement (4, 5) et des premier et second bords courts s'étendant latéralement (6, 7), **caractérisé en ce que** ; la feuille de fond (2) comprend une première partie de rabat s'étendant au moins partiellement le long du premier bord latéral (4) et repliée sur le premier bord latéral (4) et sur une partie de la feuille de dessus (3) ; la feuille de fond (2) comprend en outre une deuxième partie de rabat (9) s'étendant au moins partiellement le long du second bord latéral (5) et repliée sur le second bord latéral (5) et sur une partie de la feuille de dessus (3), dans lequel les première et deuxième parties de rabat (8, 9) sont au moins partiellement couvertes par un moyen d'adhérence (10), et la première partie de rabat (8) et la deuxième partie de rabat (9) sont fixées à la feuille de dessus (3) en au moins un point par un moyen de fixation (11), de telle sorte que des canaux (21) sont formés entre chaque partie de rabat (8, 9) et la feuille de dessus (3).

2. Article absorbant (1) selon la revendication 1, **caractérisé en ce que** le moyen d'adhérence a une force adhésive sur la peau humaine inférieure au matériau de la partie de rabat respective.

3. Article absorbant (1) selon la revendication 2, **caractérisé en ce que** le moyen d'adhérence (10) comprend un adhésif.

4. Article absorbant (1) selon l'une quelconque des revendications 1 à 3, **caractérisé en ce que** la première partie de rabat (8) et la deuxième partie de rabat (9) comprennent des parties jointes de la feuille de dessus (3) et de la feuille de fond (2).

5. Article absorbant (1) selon l'une quelconque des revendications 1 à 3, **caractérisé en ce que** la première partie de rabat (8) et la deuxième partie de rabat (9) constituent des parties indépendantes fixées à la feuille de fond (2).

6. Article absorbant (1) selon l'une quelconque des revendications précédentes, **caractérisé en ce que** l'article absorbant (1) comprend une couche absorbante (12) intercalée entre la feuille de fond (2) et la feuille de dessus (3).

7. Article absorbant (1) selon l'une quelconque des revendications précédentes, **caractérisé en ce que** l'article absorbant (1) comprend un rabat (15, 16) comprenant la première partie de rabat (8) et la deuxième partie de rabat (9) et une troisième partie de rabat (17) s'étendant le long du premier bord court latéral (6), reliant la première partie de rabat (8) à la deuxième partie de rabat (9).

8. Article absorbant (1) selon la revendication 7, **caractérisé en ce que** l'article absorbant (1) comprend un rabat (16) s'étendant le long de la périphérie de l'article absorbant en entier (1), comprenant la première partie de rabat (8), la deuxième partie de rabat (9), la troisième partie de rabat (17) et une quatrième partie de rabat (18) s'étendant le long du second bord court latéral, reliant la première partie de rabat (8) à la deuxième partie de rabat (9).

9. Procédé de fabrication d'un article absorbant (1), comprenant une feuille de fond (2) couverte au moins partiellement par une feuille de dessus (3), lequel article absorbant (1) s'étend dans une direction longitudinale et dans une direction latérale, l'article absorbant étant délimité par des premier et second bords latéraux s'étendant longitudinalement (4, 5) et des premier et second bords courts s'étendant latéralement (6, 7), dans lequel la feuille de fond (2) comprend une première partie de rabat (8) s'étendant au moins partiellement le long du premier bord latéral (4), une deuxième partie de rabat (9) s'étendant au moins partiellement le long du second bord latéral (5), où le procédé comprend les étapes consistant à ;
- intercaler la feuille de dessus (3) et la feuille de fond (2) ;
- couvrir les première et deuxième parties de rabat (8, 9) au moins partiellement avec un moyen d'adhérence (10) ;
- replier la première partie de rabat (8) sur le premier bord latéral (4) et sur une partie de la feuille de dessus (3), et ;
- replier la deuxième partie de rabat (9) sur le second bord latéral et sur une partie de la feuille de dessus.
- fixer la première partie de rabat (8) à la feuille de dessus (3) en au moins un point, en formant des canaux (21) entre la première partie de rabat (8) et la feuille de dessus (3).
- fixer la deuxième partie de rabat (9) à la feuille de dessus (3) en au moins un point, en formant des canaux (21) entre la deuxième partie de rabat (9) et la feuille de dessus (3).

10. Procédé selon la revendication 9, **caractérisé en ce que** le moyen d'adhérence a une force adhésive sur la peau humaine, inférieure au matériau de la partie de rabat respective (8, 9).

11. Procédé selon la revendication 9 ou 10, **caractérisé en ce que** des parties de la feuille de dessus (3) et de la feuille de fond (2) sont jointes en formant la première partie de rabat (8) et la deuxième partie de rabat (9).

12. Procédé selon l'une quelconque des revendications 9 à 11, **caractérisé en ce que** la première partie de rabat (8) et la deuxième partie de rabat (9) constituent des parties indépendantes fixées à la feuille de fond (2).

13. Procédé selon l'une quelconque des revendications 9 à 12, **caractérisé en ce qu'**une couche absorbante (12) est intercalée entre la feuille de fond (2) et la feuille de dessus (3).

14. Procédé selon l'une quelconque des revendications 9 à 13, **caractérisé en ce que** l'article absorbant (1) comprend un rabat (15, 16) comprenant la première partie de rabat (8) et la deuxième partie de rabat (9) et une troisième partie de rabat (17) s'étendant le long du premier bord court latéral (6), reliant la première partie de rabat (8) à la deuxième partie de rabat (9), où le rabat (15, 16) est replié sur les bords (4-6) et des parties de la feuille de dessus (3).

15. Procédé selon la revendication 14, **caractérisé en ce que** l'article absorbant (1) comprend un rabat (16) s'étendant le long de la périphérie de l'article absorbant en entier (1), comprenant la première partie de rabat (8), la deuxième partie de rabat (9), la troisième partie de rabat (17) et une quatrième partie de rabat (18) s'étendant le long du second bord court latéral, reliant la première partie de rabat (8) à la deuxième partie de rabat (9), où le rabat (16) est replié sur les bords (4-7) et des parties de la feuille de dessus (3).
